# EUROPEAN PATENT SPECIFICATION

(11) **EP 2 488 112 B1**
(45) Date of publication and mention of the grant of the patent: **30.08.2017**
(21) Application number: 10771587.2
(22) Date of filing: 06.10.2010
(51) Int. Cl.: A61B 17/17

(54) **PROTECTION SLEEVE RETENTION DEVICE**
FIXIERVORRICHTUNG FÜR SCHUTZHÜLLE
DISPOSITIF DE RETENUE DE MANCHON DE PROTECTION

(30) Priority: 15.10.2009 US 251935 P
(43) Date of publication of application: 22.08.2012
(73) Proprietor: Synthes GmbH, 4436 Oberdorf (CH)
(72) Inventor: POWELL, Sean, Coatesville Pennsylvania 19320 (US)
(74) Representative: Carpmaels & Ransford LLP
(86) International application number: PCT/US2010/051633
(87) International publication number: WO 2011/046784

(56) References cited:
- WO-A1-03/041595
- WO-A1-2006/120355
- WO-A2-2006/091625
- WO-A2-2008/086501
- DE-U1-202006 005 629
- US-A- 6 093 192

## Description

### Field of the Invention

The present invention is related to the field of bone fixation and, more particularly, related to an aiming arm configured to guide a bone fixation element to a target portion of a bone and subsequently lock the bone fixation element to the bone.

### Background

The fixation and stabilization of bones in living bodies commonly involves an implant (e.g., an intramedullary nail, etc.) inserted into a target bone. Mechanical aiming instruments are often used to aid in alignment of the implant with or within the target bone. Such an aiming arm generally comprises a protection sleeve inserted through a hole oriented towards the implant to maintain a desired position of the implant relative to the aiming arm and to provide a barrier to protect soft tissue from damage during implantation. Such protection sleeves usually also comprises an opening for the introduction of drills, screws and other instruments or implants therethrough. Presently available aiming arms provide a tensioning mechanism such as a spring, set-screw, or a spring-loaded contact element configured to tension an outer wall of the protection sleeve as it is inserted through the aiming arm. Specifically, these tensioning mechanisms apply a constant frictional force to the protection sleeve for the entire period of insertion through the aiming arm and against the implant or must be selectively engaged or disengaged by an alternate means.

WO 2006/120355 describes an arthrodetic apparatus for a first and second bone, comprising a pin, an instrument for positioning said pin comprising a base and a compression means with an operating means to produce a displacement of the pin relative to the base and an immobilisation means for the second bone relative to the base, whereby said pin comprises a guidance means.

DE 20 2006 005 629 U describes a surgical aiming device for insertion of femoral neck screws.

### Summary of the Invention

The present invention is directed to a system for inserting an implant into a bone according to claim 1. The system comprises a base defining an open central area sized to receive a portion of a patient's anatomy including a target bone into which an implant is to be inserted and a first arm coupled to the base and extending away therefrom in a first direction, a distal end of the first arm being configured to temporarily mount a proximal end of an implant thereto so that the base is in a desired orientation relative to the implant extending along a desired axis in a second direction opposite the first direction passing through the central area of the base in combination with a second arm separated laterally from the axis so that, when the first arm is in the first target orientation, the second arm extends away from the base substantially parallel to the axis in alignment with a target structure of an implant coupled to the first arm, the second arm including a first aiming hole through which the target structure is to be accessed and a protection sleeve sized for insertion through the first aiming hole through an intervening portion of soft tissue located adjacent to the bone and to the target structure of an implant coupled to the first arm, one of the protection sleeve and the first aiming hole being configured so that the protection sleeve is frictionally locked in position within the first aiming hole when the protection sleeve is rotated to a first orientation relative to the first aiming hole and, when rotated from the first orientation to a second orientation, the protection sleeve is free to move within the first aiming hole.

### Brief Description of the Drawings

Fig. 1 shows a perspective view of an exemplary system according to the present invention;
Fig. 2 shows a zoomed partial cross-sectional view of the system of Fig. 1 in an unlocked position;
Fig. 3 shows a second zoomed partial cross-sectional view of the system of Fig. 1 in a locked position;

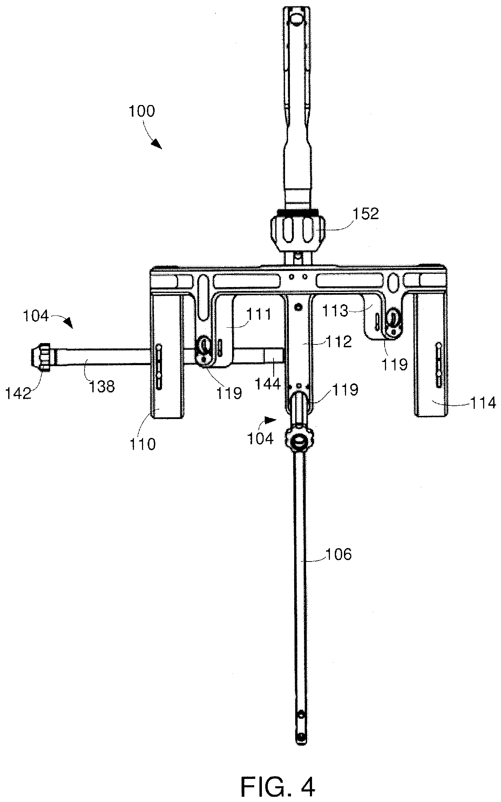

Fig. 4 shows another perspective view of the system of Fig. 1;
Fig. 5 shows another perspective view of the system of Fig. 1;
Fig. 6 shows a perspective view of a protection sleeve;
Fig. 7 shows a perspective view of a second exemplary system according to the present invention;
Fig. 8 shows a first zoomed partial cross-sectional view of the system of Fig. 7 in an unlocked position;
Fig. 9 shows a second zoomed partial cross-sectional view of the system of Fig. 7 in locked position;
Fig. 10 shows another perspective view of the system of Fig. 7; and
Fig. 11 shoes another perspective view of the system of Fig. 7.

### Detailed Description

The present invention is directed to a system for the fixation of a bone in a living body. Specifically, the present invention is directed to a system according to claim 1 comprising a protection sleeve configured for non-frictional insertion via a hole extending through an aiming arm. After the protection sleeve has been inserted to a target position relative to an implant (e.g., an intramedullary nail) positioned by the aiming arm, the protection sleeve is rotated to increase a frictional engagement between outer walls thereof with the hole through the aiming arm. Specifically, the protection sleeve is substantially cylindrical except for two flattened diametrically opposing walls extending along a portion of a longitudinal length thereof so that a width of the protection sleeve extending between the flattened walls is smaller than a diameter of outlying portions thereof. The hole formed through the aiming arm comprises leaf spring walls that are radially expandable upon application of a sufficient force thereto. In a first position, the leaf spring walls assume a cross-sectional shape substantially similar to a cross-sectional shape of the protection sleeve with a length between the leaf springs being substantially equivalent to or greater than the width of the portion of the protection sleeve extending between the flattened walls. Upon rotation of the protection sleeve about a longitudinal axis thereof, the increased diameter portions of the protection sleeve come into engagement with the leaf springs applying radially outward force thereto radially expanding the leaf spring walls. Frictional engagement between the leaf spring walls and the increased diameter outer wall of the protection sleeve then helps to maintain a desired position of the protection sleeve relative to the aiming arm. An exemplary embodiment of the present invention thus permits a physician or other user to rotationally lock and unlock the protection sleeve from the aiming arm as needed for the completion of a target bone fixation procedure. As used in this application, the term proximal refers to a direction approaching an end of the system away from a target bone hole for the insertion of a bone implant and the term distal refers to a direction approaching or located within the target bone hole. In an operative configuration, the distal end of the exemplary protection sleeve is inserted into the bone hole and inserted to a target position within the bone.

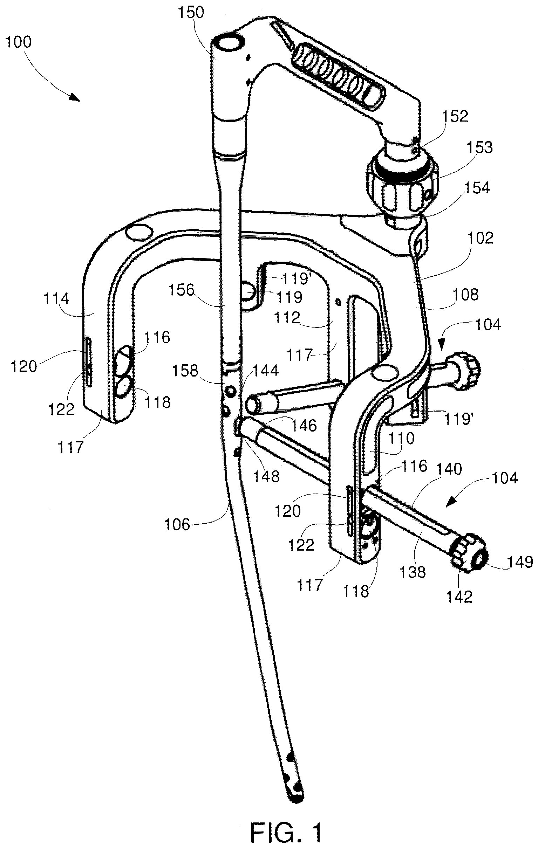

Fig. 1 shows a first exemplary system 100 according to the present invention. The system 100 comprises an aiming arm 102, a protection sleeve 104 and an intramedullary nail 106 configured for insertion into a bone (not shown) in accordance with an exemplary bone fixation procedure. The aiming arm 102 comprises a semi-circular element 108 comprising first, second third, fourth and fifth arms 110, 111, 112, 113, 114, extending distally therefrom. Each of the first, second, third, fourth and fifth arms 110, 111, 112, 113, 114 are spaced apart from one another along the semi-circular element 108, with the first and fifth arms 110, 114 located at ends of the semi-circular element 108 substantially diametrically opposed to one another. In an exemplary embodiment, the aiming arm 102 is formed of a radiolucent material (e.g., carbon fiber, plastic or aluminum). The first, second, third, fourth and fifth arms 110, 111, 112, 113, 114 extend perpendicular to a plane including the semi-circular element 108 and are substantially parallel to one another. Each of the first and fifth arms 110, 114 also comprises a combination hole 116 formed as two substantially circular holes located overlapping one another with the two circular holes open to one another, as those skilled in the art will understand, and a circular hole 118 located adjacent to the combination hole 116. A slot 120 extends into each of the combination holes 116 from a lateral wall 117 of the corresponding one of the first and fifth arms 110, 114 and extends through the combination hole 116 substantially perpendicular to axes of the holes 116, 118. A width of the slot 120 is smaller than the diameters of the circular holes making up the combination hole 116 and the circular hole 118 and a length of the slot 120 along the length of the respective one of the first and fifth arms 110, 114 is sufficient to intersect the entire length of the combination hole 116 and circular hole 118 in the same direction. The second, third and fourth arms 111, 112, 113 each only comprise a single hole 119 and a respective slot 119' configured to receive the protection sleeve 104 therethrough, as will be described in greater detail later on.

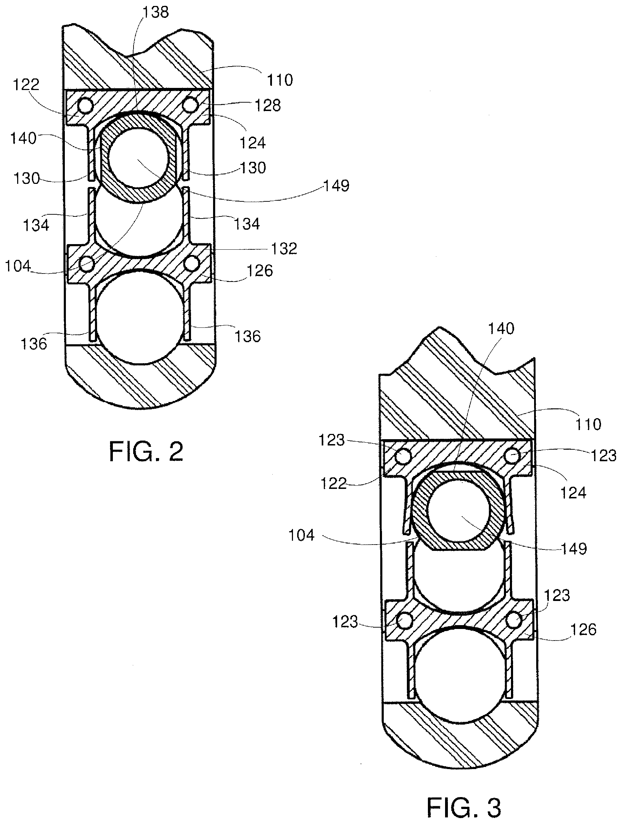

Spring elements 122 housed in each of the slots 120 comprise a first spring member 124 and a second spring member 126. The spring element 122 is formed of a sufficiently ductile material. The first and second spring members 124, 126 are not connected to one another and may optionally be employed alone if, for example, the single hole 119 is used in place of the combination hole 116. Specifically, the second, third and fourth arms 111, 112, 113 may comprise a spring element 122 positioned within the single holes 119 configured to apply a radially compressive force to any protection sleeve 104 inserted therethrough. The spring element 122 is held within the slot by one or more pins 123 forming one of a permanent and a removable connection. In an exemplary embodiment, the spring element may be formed of 302 Stainless Steel, 316 Stainless Steel, 17-7PH, Nitinol or Elgiloy ®. The first spring member 124 includes a planar element 128 having first and second leaf springs 130 extending into a first circular portion of the combination hole 116, as shown in the partial cross-sectional views of Figs. 2 - 3. The leaf springs 130 are biased to a position in which a width of the space therebetween is smaller than a diameter of a non-flattened portion of the protection sleeve 104 but greater than a width of a flattened portion thereof, as will be described in greater detail hereinafter. Similarly, the second spring member 126 is formed with a planar element 132 having a first set of leaf springs 134 extending therefrom into the combination hole 116 in a first direction and a second set of leaf springs 136 extending into the circular hole 118 in a second direction opposite the first direction. Each of the first and second sets of leaf springs 134, 136 is sized substantially similarly to the leaf springs 130 with a space formed between each pair of leaf springs being smaller than a diameter of the non-flattened portion of the protection sleeve 104 but greater than a width of the flattened portion thereof.

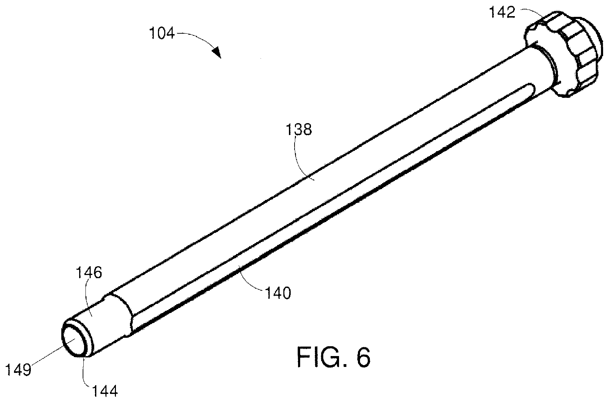

The protection sleeve 104 includes an elongated shaft 138 extending from an increased diameter head 142 with an externally scalloped shape, as those skilled in the art will understand, at a proximal end thereof to a distal end 144 having a reduced thickness portion 146. The reduced thickness portion is substantially cylindrical in shape and comprises a smaller diameter than the shaft 138, as shown in greater detail in Fig. 6. An outer surface of the shaft 138 includes a plurality of generally cylindrical portions and a plurality of flattened portions including flattened sides extending substantially parallel to one another and parallel to a longitudinal axis of the sleeve 104. Specifically, the protection sleeve 104 in this embodiment comprises two flat surfaces 140 formed on opposing diametrical sides of the shaft 138, each of the flat surfaces 140 extending along a predetermined length of the shaft, as shown in Fig. 1. The distal end 144 of the protection sleeve 104 is formed with a substantially circular cross-section having a diameter smaller than that of the shaft 138 to aid in insertion of the protection sleeve 104 into the bore 148 of the intramedullary nail 106. A channel 149 extends through the protection sleeve 104 and is open at proximal and distal ends thereof, the channel 149 having a substantially circular cross-section and being dimensioned to permit insertion of a medical instrument or implant therethrough.

The aiming arm 102 further comprises an insertion handle 150 including a first end extending away from the semi-circular element 108 substantially parallel to and opposite a direction of the arms 110 - 114. The insertion handle 150 is threadedly connected to a joint 152 and tightened therein via an adjusting knob 153 to lock a position thereof and prevent any movement of the insertion handle 150 relative to the semi-circular element 108. The insertion handle 150 extends from the joint 152 along a curved path to a second end 156 facing back toward a plane of the semi-circular element 108 and configured to engage a proximal end 158 of the intramedullary nail 106 so that the nail 106, when attached thereto, extends through the plane of the semi-circular element 108 with the bores 148 thereof aligned with the combination holes 116 and single holes 118 of the first and fifth arms 110, 114 or the single holes 119 of the second, third and fourth arms 111, 112, 113. Thus the aiming arm 102 and the insertion handle 150 hold the nail 106 in a desired position during insertion to facilitate implantation, as those skilled in the art will understand.

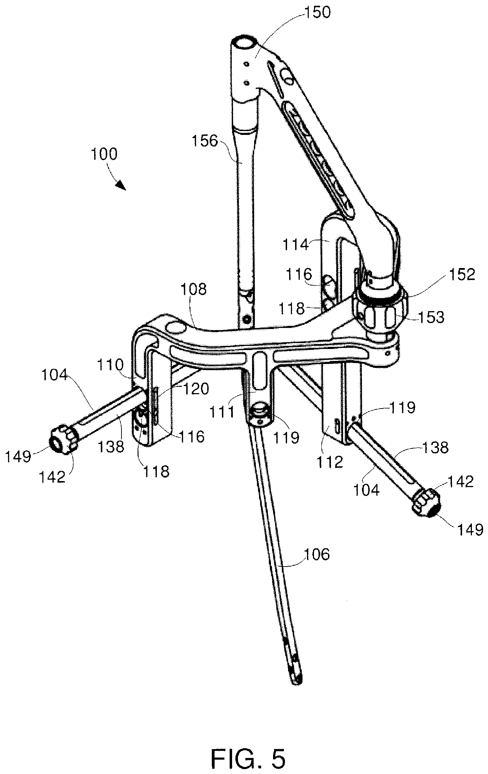

In accordance with an exemplary method, a drill (not shown) is used to drill a first bore opening to the medullary canal of a bone (not shown) so that the intramedullary nail 106 may be inserted therein. The intramedullary nail 106 is then mounted to the second end 156 of the insertion handle 150 prior to insertion thereof into the bone. A user then determines which of the arms 110 - 114 will receive the protection sleeve 104 based on the position of a fracture in the bone or a pending pathological fracture and on the geometry of the nail 106. In the provided illustration, the first arm 110 is selected to receive the protection sleeve 104. Thus, the insertion handle 150 and the intramedullary nail 106 are oriented so that, when the intramedullary nail 106 is inserted to a desired position in the first bore (not shown), a drill inserted through the protection sleeve 104 and through the combination hole 116 or the circular hole 118 of the first arm 110 is in alignment with the bore 148 extending through the intramedullary nail 106. As shown in Figs. 1 and 5, the intramedullary nail 106 comprises a plurality of additional bores 148' extending therethrough at a plurality of angles, each of the bores 148' being substantially perpendicular to a longitudinal axis of the intramedullary nail 106. Accordingly, a user of the system 100 may select more than one of the arms 110 - 114 to receive the protection sleeve 104 therethrough. Thus, the intramedullary nail 106 may receive any plurality of protection sleeves 104. The intramedullary nail 106 is then inserted into the bone and the protection sleeve 104 is inserted into the combination hole 116 in a first configuration with the flat surfaces 140 aligned with the leaf springs 130 so that the protective sleeve 104 slides therepast with a minimal amount of resistance. Once the protection sleeve 104 has been inserted to the target depth in the bore 148, the protection sleeve 104 is rotated by approximately 90 degrees so that the portions of the shaft 138 having an increased diameter relative to the flat surfaces 140 are in contact with the leaf springs 130 deflecting the leaf springs 130 radially outward against the spring bias and frictionally engaging the leaf springs 130 with the substantially cylindrical portions of the shaft 138 locking the protection sleeve 104 in the desired position. Once the protection sleeve 104 has been locked in this desired position, a drill, screw or other instrument or implant may be inserted through the channel 149 into the bore 148 to drill a transverse second bore at an angle to the first bore (not shown). As those skilled in the art will understand, the second bore extends through a lateral cortex of the bone at a point selected to align with the bore 148 when the nail 106 is in a desired position within the bone.

The protection sleeve 104 is formed with a substantially circular cross-section having two flat surfaces 140 formed on opposing walls thereof. In a first alternate embodiment of the present invention, the shaft 138 may be formed with any cross-sectional shape, including, but not limited to square, hexagonal, octagonal, or another polygon shape so long as the cross-sectional geometry of the sleeve 104 relative to the leaf springs 130 such that, in a first orientation, a reduced diameter or reduced width aspect is presented to the leaf springs 130 and, in a second orientation, a larger diameter or width portion is presented to the leaf springs 130 deflecting the leaf springs 130 radially outward and increasing a resistance to the movement of the protection sleeve 104 relative to the one of the arms 110 - 114 through which it is inserted. The protection sleeve 104 may further comprise any type of recesses and is not limited to the flat surfaces 140. Specifically, the recesses may be concave, convex, helical or any other shape wherein an outer diameter of the recess is smaller than an outer diameter of the shaft 138. Furthermore, the protection sleeve 104 may comprise one or any plurality of recesses formed along an outer wall thereof. In yet another embodiment, the recesses may be replaced by features having the same outer diameter as the shaft 138 but which comprise a different material than the shaft 138 such as Nylon or Teflon or may comprise an alternate surface finish (e.g., knurling, grooving or threading) with properties selected to reduce or enhance frictional engagement of portions of the outer surface with the leaf springs 130 relative to the frictional engagement of a surface finish of other portions of the outer surface of the shaft 138.

In another embodiment of the present invention, the aiming arm 102 may comprise one or more leaf springs 130. Furthermore, the leaf springs 130 may be replaced with coil springs, thin flexible mechanical elements (e.g., belleville washers), wave spring, compressible cylinders, wave washers or portions of elastic material (e.g., rubber, plastic). The leaf springs 130 may be intrinsic with the arms 110 - 114 or, in an alternate embodiment, a separate element may be used to enhance contact therewith. The planar element 128 may be permanently affixed to the slot 120 by use of a pin or other mechanical attachment mechanism known in the art or, in an alternate embodiment, may be removable therefrom as needed.

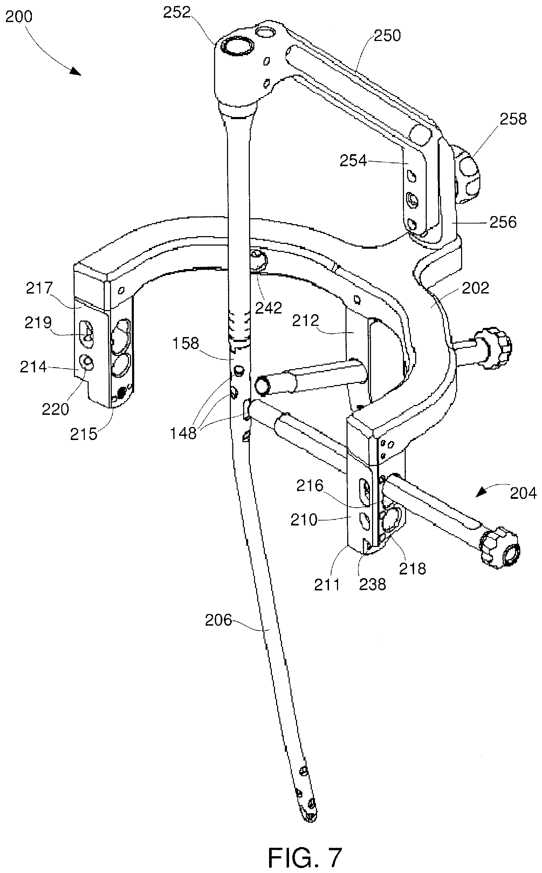

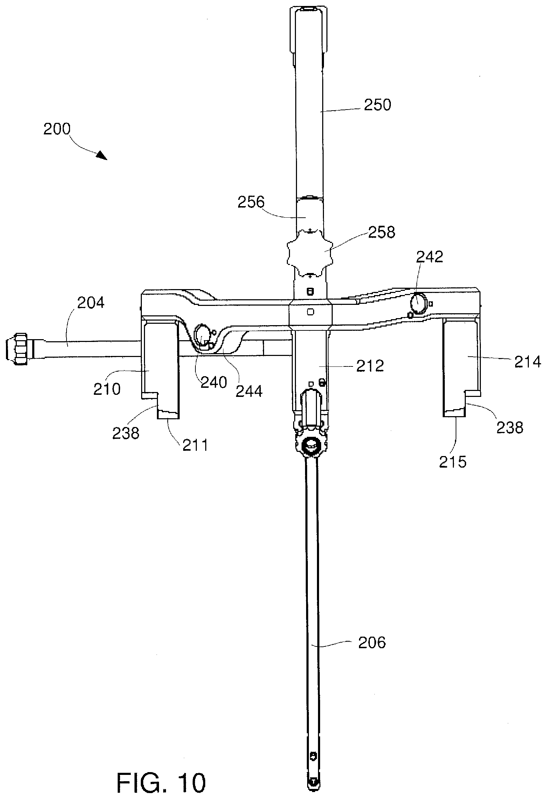

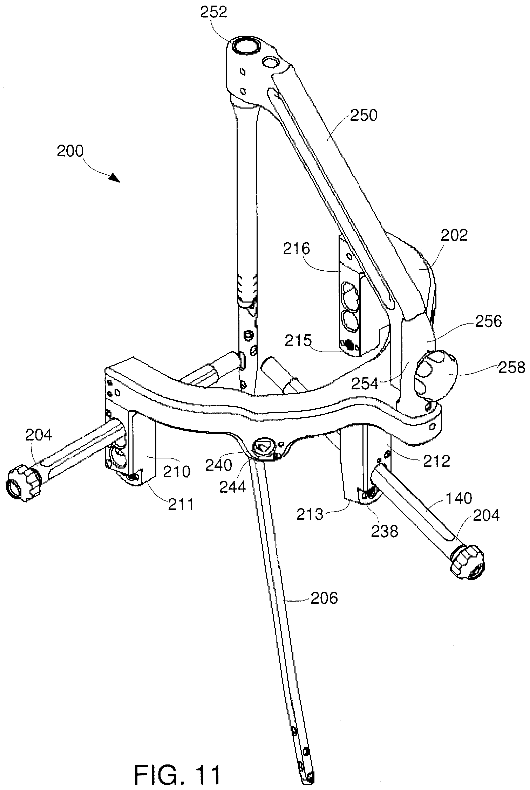

Figs. 7-11 depict a system 200 according to another exemplary embodiment of the present invention. The system 200 is formed substantially similarly to the system 100, with like elements referenced by like reference numerals. The system 200 comprises an aiming arm 202, a protection sleeve 204 and an intramedullary nail 206 formed substantially similarly to the respective elements of system 100. A semi-circular element 208 of the aiming arm 202 comprises first, second and third arms 210, 212, 214 extending distally therefrom and spaced apart from one another. In an exemplary embodiment, the first, second and third arms 210, 212, 214 extend substantially perpendicularly from a plane housing the aiming arm 202 and are substantially parallel to one another. Similar to the system 100, each of the first, second and third arms 210, 212, 214 comprises a combination hole 216 extending laterally therethrough and a circular hole 218 adjacent thereto. Instead of a slot 120 as taught in system 100, the system 200 comprises a lateral, substantially oval hole 219 and a lateral circular hole 220 extending through a lateral wall 217 thereof. Each of the lateral holes 219, 220 have a length corresponding to the length of the combination hole 216 and circular hole 218, respectively. In an exemplary embodiment, the lateral oval hole 219 and the lateral circular hole 220 are configured and dimensioned so that spring elements 222, 224, 226 inserted therethrough project into at least a portion of the combination hole 216 and the circular hole 218, respectively, as will be described in greater detail hereinafter.

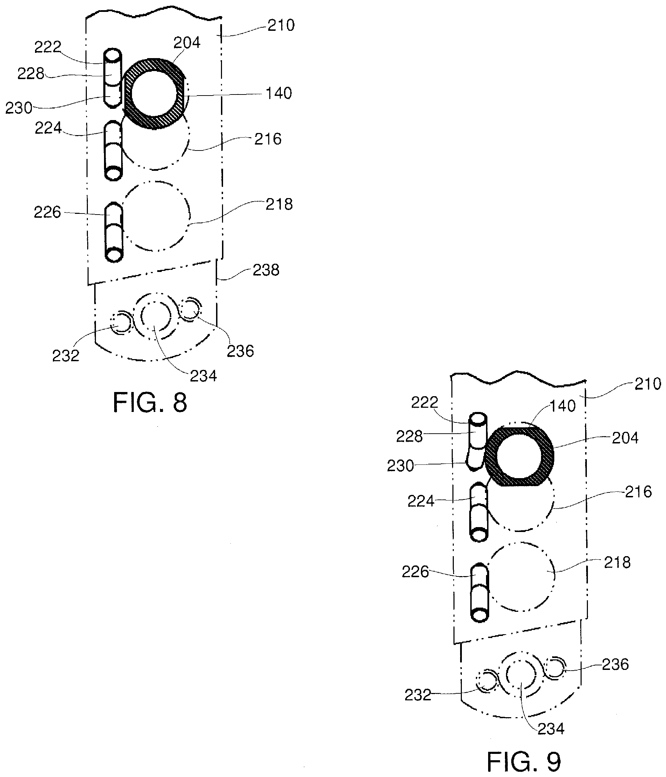

Exemplary spring elements 222, 224, 226 according to the exemplary embodiment are configured as substantially planar elements formed of a material substantially similar to a material of the spring elements 122 of system 100. Each of the spring elements 222, 224 and 226 is separate from the others and is configured for separate, permanent insertion into one of the lateral holes 219, 220. The spring elements 222, 224, 226 are held in place within respective ones of the lateral holes 219, 220 by a friction fit (e.g., during manufacturing). Each of the spring elements 222, 224, 226 comprises a first portion 228 and a second portion 230, the second portion 230 being configured and dimensioned to deflect laterally away from a center of the combination hole 216 or circular hole 218 upon application of a pressure thereto (e.g., by rotation of the protection sleeve 204), as described in greater detail with respect to the system 100. Each of the spring elements 222, 224, 226 is positioned to project partially into the combination hole 216 and circular hole 218 by a length substantially equivalent to a depth of a cutout formed in the protection sleeve forming the flattened portion 140, as also described in greater detail earlier. This configuration permits the spring elements to remain substantially unobstructed when the protection sleeve 204 is inserted into the combination hole 216 in the configuration shown in Fig. 8. Upon rotation of the protection sleeve 204 to the position shown in Fig. 9, a radially expansive force is applied to the spring element 222 causing the second portion 230 to deflect radially outward.

As shown in Figs. 8 and 9, each of the first, second and third arms 210, 212, 214 also comprises additional locking holes 232, 234, 236 adjacent respective ends 211, 213, 215 thereof. The additional locking holes 232, 234, 236 may be used to attached extension pieces (not shown) to the system 100. The ends 211, 213, 215 also comprise stepped portions 238 having a thickness reduced relative to proximal portions of the arms 210, 212, 214 to permit attachment to the extension pieces (not shown).

The exemplary embodiment of system 200 obviates the need for additional arms disposed between the first, second and third arms 210, 212, 214, instead replacing these arms with first and second sleeve holes 240, 242. Specifically, the first sleeve hole 240 is provided on a portion of the aiming arm 202 substantially equidistant from the first arm 210 and the second arm 214. A partially circular extension portion 244 of the aiming arm 202 extends distally from the aiming arm 202 and along a curve having a radius of curvature suited to the dimensions of the sleeve hole 240, as those skilled in the art will understand. In an exemplary embodiment, the position of the extension portion 244 and the sleeve hole 242 are selected so that a drill sleeve inserted through the sleeve hole 242 is aligned with an opening extending through the intramedullary nail 206. It is therefore noted that the dimensions of the extension portion 244 may be varied to suit the requirements of a predetermined procedure. Similarly, the sleeve hole 242 positioned between the second and third arms 212, 214 may be positioned elsewhere along the aiming arm to conform to the requirements of an intramedullary nail 206 to be used therewith. In the embodiment shown, a portion of the aiming arm 202 housing the sleeve hole 242 is longitudinally offset from the plane housing the aiming arm 202. It is respectfully submitted that the aiming arm 202 may be formed with any geometry to permit a drill sleeve 204 inserted therethrough to intersect with a target portion of the intramedullary nail 206 without deviating from the scope of the present invention.

The system 200 further comprises an insertion handle 250 formed substantially similarly to the insertion handle 150 of the system 100. Specifically, the insertion handle 250 extends away from the aiming arm 202 along a curved path in a direction substantially opposite a direction of the arms 210, 212, 214 to an end 252 facing back toward the plane of the aiming arm 202. A locking portion 254 of the insertion handle 250 is configured for locking engagement with a locking portion 256 of the aiming arm 202, by, for example, tightening of a threaded knob 258 through each of the locking portions 254, 256. Tightening of the knob 258 locks a position of the insertion handle 250 relative to the aiming arm 202. The end 252 is configured to engage the proximal end 158 of the intramedullary nail 206 so that the nail 106, when attached thereto, extends through the plane of the aiming arm 202 with bores 148 thereof aligned with at least one of the combination holes 216, circular holes 218 or sleeve holes 240, 242. Thus the aiming arm 202 and the insertion handle 250 hold the nail 206 in a desired position during insertion to facilitate implantation. The present invention has been described with respect to intramedullary nails for the fixation of long bones. It is noted however, that the exemplary system of the present invention may also be employed in securing protection sleeves in aiming arms for surgical bone plates or artificial joints. Furthermore, the exemplary system of the present invention may also be used to secure a cylindrical instrument known in the art (e.g., a drill sleeve) into a hollow portion of another cylindrical instrument known in the art (e.g., a protection sleeve).

Although the present invention has been described with reference to preferred embodiments, it is submitted that various modifications can be made to the exemplary system without departing from the scope of the invention, as defined by the appended claims.

## Claims

1. A system (100) for inserting an implant into a bone, comprising:
a base (108) defining an open central area sized to receive a portion of a patient's anatomy including a target bone into which an implant is to be inserted;
a first arm (112) coupled to the base (108) and extending away therefrom in a first direction, a distal end of the first arm (112) being configured to removably mount a proximal end of an implant thereto so that the base (108) is in a desired orientation relative to the implant extending along a desired axis in a second direction opposite the first direction passing through the central area of the base (108);
a second arm (110) separated laterally from the axis so that the second arm (110) extends away from the base (108) substantially parallel to the axis in alignment with a target structure of an implant coupled to the first arm (112), the second arm (110) including a first aiming hole (116) through which the target structure is to be accessed, the first aiming hole (116) including a first spring element (122) extending thereinto; and
a first protection sleeve (104) sized for insertion through the first aiming hole (116) through an intervening portion of soft tissue located adjacent to the target structure of an implant coupled to the first arm (112),
wherein one of the first protection sleeve (104) and the first aiming hole (116) is configured so that the first spring element (122) engages the first protection sleeve (104) to frictionally lock the first protection sleeve in position within the first aiming hole (116) when the first protection sleeve (104) is rotated to a first orientation relative to the first aiming hole (116) and, when rotated from the first orientation to a second orientation, the first spring element (122) disengages the first protection sleeve (104) so that the first protection sleeve is free to move within the first aiming hole (116), and
wherein the first protection sleeve (104) includes a first reduced diameter portion which, when rotated to face the first spring element (122), places the first protection sleeve (104) in the second orientation relative to the first aiming hole (116), and rotation of the first protection sleeve (104) so that a second increased diameter portion thereof engages the first spring element (122) defining the first orientation so that the first spring element (122) locks the increased diameter portion of the first protection sleeve (104) within the first aiming hole (116).

2. The system of claim 1, wherein the first and second portions of the first protection sleeve (104) are separated from one another around a circumference of the first protection sleeve (104).

3. The system of claim 1, wherein the first protection sleeve (104) includes a first portion including surface features increasing a frictional engagement with an inner wall of the first aiming hole (116) in comparison with a frictional engagement between a second portion of the first protection sleeve including a surface (140) adapted to minimize frictional engagement with the inner wall of the first aiming hole (116).

4. The system of claim 3, wherein the first and second portions of the first protection sleeve (104) are separated from one another around a circumference of the first protection sleeve.

5. The system of claim 1, wherein the base (108) is substantially C-shaped with the first arm (112) mounted at a central portion thereof and the second arm (110) mounted at an end thereof.

6. The system of claim 1, further comprising a third arm (114) separated laterally from the axis on a side of the open central area of the base (108) opposite the second arm (110), the third arm (114) being positioned to extend away from the base (108) substantially parallel to the axis in alignment with a target structure of an implant coupled to the first arm (112), the third arm (114) including a second aiming hole (116) through which the target structure is to be accessed.

7. The system of claim 6, wherein the second hole (116) is a combination hole.

8. The system of claim 1, wherein the first spring element (122) is permanently affixed to the first arm (112).

9. The system of claim 1, wherein the first spring element (122) is removably affixed to the first arm (112).

10. The system of claim 1, wherein the first spring element (122) is one of a leaf spring, coil spring, belleville washer, compressible cylinder, wave spring and wave washer.

11. The system of claim 6, further comprising a fourth arm (111) located between the second arm (110) and the third arm (114), the fourth arm (111) extending substantially parallel to the axis in alignment with the target structure of the implant and including a third aiming hole (116) through which the target structure is to be accessed, the third aiming hole (116) comprising a substantially circular cross-section.

12. The system of claim 11, further comprising a second protection sleeve (104) sized for insertion through a selected one of the second and third aiming holes (116) through an intervening portion of soft tissue located adjacent to a second target structure of the implant coupled to the first arm (112), one of the second protection sleeve (104) and the selected one of the second and third aiming holes (116) being configured so that the second protection sleeve (104) is frictionally locked in position therewithin when the second protection sleeve (104) is rotated to a third orientation relative to the selected aiming hole (116) and, when rotated from the third orientation to a fourth orientation, the second protection sleeve (104) is free to move within the selected aiming hole (116).

## Patentansprüche

1. System (100) zur Einführung eines Implantats in einen Knochen, umfassend:
eine Basis (108), die einen offenen mittleren Bereich mit einer Größe zur Aufnahme eines Teils der Anatomie eines Patienten, einschließlich eines Zielknochens, in den ein Implantat eingeführt werden soll, definiert;
einen ersten Arm (112), der mit der Basis (108) verbunden ist und sich von dieser in einer ersten Richtung weg erstreckt, wobei ein distales Ende des ersten Arms (112) dazu ausgelegt ist, ein proximales Ende eines Implantat entfernbar daran zu befestigen, derart, dass sich die Basis (108) in einer gewünschten Orientierung bezüglich des Implantats befindet, die sich entlang einer gewünschten Achse in eine zweite Richtung erstreckt, die der ersten Richtung entgegengesetzt ist und durch den mittleren Bereich der Basis (108) verläuft;
einen zweiten Arm (110), der seitlich von der Achse getrennt ist, derart, dass sich der zweite Arm (110) von der Basis (108) weg im Wesentlichen parallel zur Achse in Ausrichtung mit einer Zielstruktur eines mit dem ersten Arm (112) verbundenen Implantats erstreckt, wobei der zweite Arm (110) ein erstes Zielloch (116) aufweist, durch das auf die Zielstruktur zugegriffen werden soll, wobei das erste Zielloch (116) ein erstes Federelement (122) aufweist, das sich dort hinein erstreckt; und
eine erste Schutzhülle (104) mit einer Größe zur Einführung durch das erste Zielloch (116) durch einen intervenierenden Abschnitt von Weichgewebe neben der Zielstruktur eines mit dem ersten Arm (112) verbundenen Implantats,
wobei die erste Schutzhülle (104) oder das erste Zielloch (116) derart ausgelegt ist, dass das erste Federelement (122) die erste Schutzhülle (104) in Eingriff nimmt, um die erste Schutzhülle reibschlüssig in ihrer Position im ersten Zielloch (116) zu verriegeln, wenn die erste Schutzhülle (104) in eine erste Orientierung bezüglich des ersten Ziellochs (116) gedreht wird, und wobei sich das erste Federelement (122) bei der Drehung aus der ersten Orientierung in eine zweite Orientierung aus seinem Eingriff mit der ersten Schutzhülle (104) löst, derart, dass sich die erste Schutzhülle frei im ersten Zielloch (116) bewegen kann, und
wobei die erste Schutzhülle (104) einen ersten Abschnitt mit reduziertem Durchmesser aufweist, der, wenn er so gedreht wird, dass er dem ersten Federelement (122) zugewandt ist, die erste Schutzhülle (104) in die zweite Orientierung bezüglich des ersten Ziellochs (116) platziert, und eine Drehung der ersten Schutzhülle (104), derart, dass ein zweiter Abschnitt davon mit vergrößertem Durchmesser das erste Federelement (122) in Eingriff nimmt, die erste Orientierung definiert, derart, dass das erste Federelement (122) den Abschnitt mit vergrößertem Durchmesser der ersten Schutzhülle (104) im ersten Zielloch (116) verriegelt.

2. System nach Anspruch 1, wobei die ersten und zweiten Abschnitte der ersten Schutzhülle (104) um einen Umfang der ersten Schutzhülle (104) voneinander getrennt sind.

3. System nach Anspruch 1, wobei die erste Schutzhülle (104) einen ersten Abschnitt aufweist, der Oberflächenmerkmale besitzt, die einen reibschlüssigen Eingriff mit einer Innenwand des ersten Ziellochs (116) im Vergleich zu einem reibschlüssigen Eingriff zwischen einem zweiten Abschnitt der ersten Schutzhülle mit einer Oberfläche (140), die zur Minimierung eines reibschlüssigen Eingriffs mit der Innenwand des ersten Ziellochs (116) ausgelegt ist, erhöhen.

4. System nach Anspruch 3, wobei die ersten und zweiten Abschnitte der ersten Schutzhülle (104) um einen Umfang der ersten Schutzhülle voneinander getrennt sind.

5. System nach Anspruch 1, wobei die Basis (108) im Wesentlichen C-förmig ist, wobei der erste Arm (112) an einem mittleren Abschnitt davon befestigt ist und der zweite Arm (110) an einem Ende davon befestigt ist.

6. System nach Anspruch 1, ferner umfassend einen dritten Arm (114), der seitlich von der Achse auf einer Seite des offenen mittleren Bereichs der Basis (108) gegenüber dem zweiten Arm (110) getrennt ist, wobei der dritte Arm (114) derart platziert ist, dass er sich von der Basis (108) weg im Wesentlichen parallel zur Achse in Ausrichtung mit einer Zielstruktur eines mit dem ersten Arm (112) verbundenen Implantats erstreckt, wobei der dritte Arm (114) ein zweites Zielloch (116) aufweist, durch das auf die Zielstruktur zugegriffen werden soll.

7. System nach Anspruch 6, wobei das zweite Loch (116) ein Kombinationsloch ist.

8. System nach Anspruch 1, wobei das erste Federelement (122) permanent am ersten Arm (112) befestigt ist.

9. System nach Anspruch 1, wobei das erste Federelement (122) entfernbar am ersten Arm (112) befestigt ist.

10. System nach Anspruch 1, wobei das erste Federelement (122) eine Blattfeder, eine Spiralfeder, eine Tellerfeder, ein komprimierbarer Zylinder, eine Wellfeder oder ein Federring ist.

11. System nach Anspruch 6, ferner umfassend einen vierten Arm (111), der zwischen dem zweiten Arm (110) und dem dritten Arm (114) angeordnet ist, wobei sich der vierte Arm (111) im Wesentlichen parallel zur Achse in Ausrichtung mit der Zielstruktur des Implantats erstreckt und ein drittes Zielloch (116) aufweist, durch das auf die Zielstruktur zugegriffen werden soll, wobei das dritte Zielloch (116) einen im Wesentlichen kreisförmigen Querschnitt umfasst.

12. System nach Anspruch 11, ferner umfassend eine zweite Schutzhülle (104) mit einer Größe zur Einführung durch ein ausgewähltes der zweiten und dritten Ziellöcher (116) durch einen intervenierenden Abschnitt von Weichgewebe neben einer zweiten Zielstruktur des mit dem ersten Arm (112) verbundenen Implantats, wobei die zweite Schutzhülle (104) oder das ausgewählte der zweiten und dritten Ziellöcher (116) derart ausgelegt ist, dass die zweite Schutzhülle (104) darin reibschlüssig in ihrer Position verriegelt wird, wenn die zweite Schutzhülle (104) in eine dritte Orientierung bezüglich des ausgewählten Ziellochs (116) gedreht wird, und wobei sich die zweite Schutzhülle (104) bei der Drehung aus der dritten Orientierung in eine vierte Orientierung frei im ausgewählten Zielloch (116) bewegen kann.

## Revendications

1. Système (100) pour insérer un implant dans un os, comprenant:
une base (108) définissant une région centrale ouverte dimensionnée de manière à recevoir une partie de l'anatomie d'un patient comprenant un os cible dans lequel un implant doit être inséré;
un premier bras (112) couplé à la base (108) et s'étendant à l'écart de celle-ci dans une première direction, une extrémité distale du premier bras (112) étant configurée de manière à monter de façon amovible une extrémité proximale d'un implant sur celle-ci de telle sorte que la base (108) se trouve dans une orientation souhaitée par rapport à l'implant s'étendant le long d'un axe souhaité dans une seconde direction opposée à la première direction et passant à travers la région centrale de la base (108);
un deuxième bras (110) séparé latéralement de l'axe de telle sorte que le deuxième bras (110) s'étende à l'écart de la base (108) de façon sensiblement parallèle à l'axe en alignement avec une structure cible d'un implant couplé au premier bras (112), le deuxième bras (110) comprenant un premier trou de visée (116) à travers lequel il est possible d'accéder à la structure cible, le premier trou de visée (116) comprenant un premier élément de ressort (122) qui s'étend à travers celui-ci; et
un premier manchon de protection (104) dimensionné de manière à être inséré à travers le premier trou de visée (116) à travers une partie intermédiaire de tissu mou située à proximité de la structure cible d'un implant couplé au premier bras (112),
dans lequel un parmi le premier manchon de protection (104) et le premier trou de visée (116) est configuré de telle manière que le premier élément de ressort (122) engage le premier manchon de protection (104) afin de verrouiller par friction le premier manchon de protection en position à l'intérieur du premier trou de visée (116) lorsque le premier manchon de protection (104) est tourné dans une première orientation par rapport au premier trou de visée (116) et, lorsqu'il est tourné depuis la première orientation dans une deuxième orientation, le premier élément de ressort (122) désengage le premier manchon de protection (104) de telle sorte que le premier manchon de protection soit libre de se déplacer à l'intérieur du premier trou de visée (116), et
dans lequel le premier manchon de protection (104) comprend une première partie de diamètre réduit qui, lorsqu'elle est tournée pour faire face au premier élément de ressort (122), place le premier manchon de protection (104) dans la deuxième orientation par rapport au premier trou de visée (116), et fait tourner le premier manchon de protection (104) de telle sorte qu'une seconde partie de diamètre accru de celui-ci engage le premier élément de ressort (122), définissant la première orientation de telle sorte que le premier élément de ressort (122) verrouille la partie de diamètre accru du premier manchon de protection (104) à l'intérieur du premier trou de visée (116).

2. Système selon la revendication 1, dans lequel les première et seconde parties du premier manchon de protection (104) sont séparées l'une de l'autre autour d'une circonférence du premier manchon de protection (104).

3. Système selon la revendication 1, dans lequel le premier manchon de protection (104) comprend une première partie présentant des caractéristiques de surface qui augmentent un engagement par friction avec une paroi intérieure du premier trou de visée (116) comparativement à un engagement par friction entre une seconde partie du premier manchon de protection présentant une surface (140) apte à minimiser un engagement par friction avec la paroi intérieure du premier trou de visée (116).

4. Système selon la revendication 3, dans lequel les première et seconde parties du premier manchon de protection (104) sont séparées l'une de l'autre autour d'une circonférence du premier manchon de protection.

5. Système selon la revendication 1, dans lequel la base (108) est sensiblement en forme de C avec le premier bras (112) monté à une partie centrale de celle-ci et le deuxième bras (110) monté à une extrémité de celle-ci.

6. Système selon la revendication 1, comprenant en outre un troisième bras (114) séparé latéralement de l'axe sur un côté de la région centrale ouverte de la base (108) opposé au deuxième bras (110), le troisième bras (114) étant positionné de manière à s'étendre à l'écart de la base (108) sensiblement parallèlement à l'axe en alignement avec une structure cible d'un implant couplé au premier bras (112), le troisième bras (114) comportant un deuxième trou de visée (116) à travers lequel il est possible d'accéder à la structure cible.

7. Système selon la revendication 6, dans lequel le deuxième trou (116) est un trou de combinaison.

8. Système selon la revendication 1, dans lequel le premier élément de ressort (122) est fixé de façon permanente au premier bras (112).

9. Système selon la revendication 1, dans lequel le premier élément de ressort (122) est fixé de façon amovible au premier bras (112).

10. Système selon la revendication 1, dans lequel le premier élément de ressort (122) est soit un ressort à lame, un ressort hélicoïdal, une rondelle Belleville, un cylindre compressible, un ressort ondulé ou une rondelle ondulée.

11. Système selon la revendication 6, comprenant en outre un quatrième bras (111) qui est situé entre le deuxième bras (110) et le troisième bras (114), le quatrième bras (111) s'étendant sensiblement parallèlement à l'axe en alignement avec la structure cible de l'implant et comportant un troisième trou de visée (116) à travers lequel il est possible d'accéder à la structure cible, le troisième trou de visée (116) présentant un section transversale sensiblement circulaire.

12. Système selon la revendication 11, comprenant en outre un second manchon de protection (104) dimensionné de manière à être inséré à travers un trou sélectionné parmi les deuxième et troisième trous de visée (116) à travers une partie intermédiaire de tissu mou située à proximité d'une seconde structure cible de l'implant couplé au premier bras (112), un parmi le second manchon de protection (104) et le trou sélectionné parmi les second et troisième trous de visée (116) étant configuré de telle manière que le second manchon de protection (104) soi verrouillé par friction en position à l'intérieur de celui-ci lorsque le second manchon de protection (104) est tourné dans une troisième orientation par rapport au trou de visée sélectionné (116) et, lorsqu'il est tourné depuis la troisième orientation dans une quatrième orientation, le second manchon de protection (104) est libre de se déplacer à l'intérieur du trou de visée sélectionné (116).
